# EUROPÄISCHE PATENTSCHRIFT

(11) **EP 3 221 147 B1**
(45) Veröffentlichungstag und Bekanntmachung des Hinweises auf die Patenterteilung: **21.06.2023**
(21) Anmeldenummer: 15808357.6
(22) Anmeldetag: 19.11.2015
(51) Int. Cl.: B32B 29/00, E04B 1/94, B32B 5/02, B32B 5/22, B32B 5/24, B32B 19/00, B32B 19/04, B32B 19/06, B32B 29/02

(54) **NICHT BRENNBARER SCHICHTSTOFF**
NON-FLAMMABLE LAMINATE
MATÉRIAU STRATIFIÉ ININFLAMMABLE

(30) Priorität: 20.11.2014 DE 102014116984
(43) Veröffentlichungstag der Anmeldung: 27.09.2017
(73) Patentinhaber: DI Dekodur International GmbH & Co. KG, 69434 Hirschhorn/Neckar (DE)
(72) Erfinder: ANDRÉ, Volkmar, 69434 Hirschhorn (DE); HELD, Holger, 64711 Erbach im Odenwald (DE)
(74) Vertreter: Weisbrodt, Bernd
(86) Internationale Anmeldenummer: PCT/EP2015/077117
(87) Internationale Veröffentlichungsnummer: WO 2016/079245

(56) Entgegenhaltungen:
- EP-A1- 0 899 092
- DE-A1- 2 907 707
- DE-A1-102007 037 137
- US-A- 3 736 220

## Beschreibung

Die Erfindung betrifft einen Schichtstoff, insbesondere zur Herstellung einer Baustoffplatte mit den Merkmalen des Oberbegriffs des Anspruchs 1.

Es ist aus dem Stand der Technik bekannt, Platten aus Schichtstoffen im Innen- und Außenbereich einzusetzen. An derartige Platten werden je nach Anwendungsbereich verschiedene Anforderungen gestellt. Beispielsweise werden Schichtstoffplatten mit dekorativ gestalteter Oberfläche in der Möbelindustrie, im Haushaltsbereich, an öffentlichen Orten und in vielen sonstigen Einsatzgebieten verwendet.

Zur Herstellung dekorativer Schichtstoffplatten wird ein Schichtaufbau vorgenommen, bei welchem zunächst eine oder mehrere Lagen mit Kunstharz getränkter Träger auf eine stabile Platte gelegt werden, welche später in eine Plattenpresse zur Verpressung mit einem Druck größer 5 MPa verbracht wird. Dabei können auch anorganische Träger wie Vliesstoffe, beispielsweise aus Glas- oder Mineralfasern bestehend, verwendet werden.

Das zur Imprägnierung des Grundkörpers verwendete Harz ist normalerweise ein duroplastisch härtbares Harz, wie beispielsweise ein Phenolformaldehydharz, wobei jedoch auch aus technischer Sicht andere duroplatisch aushärtbare Vorkondensate wie Melamin- oder Harnstoffharze als auch Harze auf der Basis von Naturstoffen geeignet sind.

Auf den mit einem Harz imprägnierten Grundkörper können eine oder auch mehrere Lagen von Papier aufgelegt werden, welche ebenfalls mit einem Harz, wie beispielsweise Melaminharz, getränkt sind. Diese Lagen aus Papier bilden die oberste Schicht einer Schichtstoffplatte und werden auch als Dekorschicht bezeichnet.

Als Träger für die Dekorschicht wird normalerweise reines Zellulosepapier eingesetzt, welches mittels einer Pigmentierung allein wirkt oder zusätzlich bedruckt und/oder gefärbt ist und mit dem harzgetränkten Grundkörper in einer beheizbaren Presse unter Temperatur und hohem Flächendruck unter entsprechender Druck- und Wärmeeinwirkung miteinander verpresst wird.

Für die Dekorschicht kommt als Bindemittel ein möglichst farbloses Kunstharz zum Einsatz, vorzugsweise aufgrund seiner langen Haltbarkeit ein Melaminformaldehydharz. Ferner kommt auch die Verwendung von Harnstoffharzen oder Melamin-Harnstoff-Mischharzen zur Imprägnierung der Dekorschicht in Frage. Prinzipiell können auf der Dekorschicht weitere Schichten angeordnet sein, welche beispielsweise zur Verbesserung der Verschleißfestigkeit der Oberfläche zum Erzielen optischer Effekte oder sonstigen Verwendungszwecken, wie beispielsweise dem Brandschutz, eingesetzt werden. Um Anforderungen an den Brandschutz gerecht zu werden, ist aus der DE 10 2007 037 137 A1 eine Platte bekannt, welche aus einer Glasfaserplatte und einer darauf angeordneten Platte aus schwer entflammbarem oder unbrennbarem Material besteht. Nachteilig an der aus dem Stand der Technik bekannten Platte ist insbesondere die hohe Gesamtdicke des Verbundes, welche 5 mm oder mehr beträgt. Besonders bei Verwendung einer solchen als dekorative Platte ausgebildeten Platte ist es nötig, eine geringe Gesamtdicke zu erzielen, um ästhetischen Anforderungen genügen zu können. Des Weiteren bestehen jedoch nach wie vor die teils gesetzlich vorgeschriebenen Anforderungen hinsichtlich des Brandschutzes. EP0 899 092 A1 offenbart eine flammenhemmende oder nichtentflammbare Dekorlage, die zwei oder mehrere Schichten eines Flächengebildes für eine Kernschicht enthält, das erhalten wird durch Tränken eines Basis-Flächengebildes, das aus einem Faservlies aus anorganischen Fasern hergestellt ist, mit einer Zusammensetzung, die ein Phenolharz und/oder ein Melaminharz sowie Aluminiumhydroxid und/oder Magnesiumhydroxid umfasst, und ein Flächengebilde für eine Dekorschicht, das auf mindestens einer Seite des Flächengebildes für die Kernschicht aufgebracht ist. Die Gesamtdicke der Dekorlage beträgt bevorzugt 3 mm oder mehr.

Vor diesem Hintergrund liegt der vorliegenden Erfindung die Aufgabe zugrunde, einen verbesserten Schichtstoff bereitzustellen, welcher insbesondere eine geringe Gesamtdicke aufweist und ferner insbesondere in Bezug auf die Brandschutzklasse A1 gemäß DIN 4102-1 beziehungsweise EN 13501 ausreichende Brandschutzeigenschaften bereitstellt.

Zur technischen Lösung wird mit der vorliegenden Erfindung ein Schichtstoff, mit den Merkmalen des Anspruchs 1 vorgeschlagen.

Die Erfindung macht dabei insbesondere von der Erkenntnis Gebrauch, dass im Rahmen der Fertigung beziehungsweise Herstellung eines erfindungsgemäßen Schichtstoffes aufgrund der Kombination einer aus einem nicht brennbaren, mit einem Kunstharz imprägnierten Vliesstoff mit einer Dekormaterialschicht aus einem mit Melaminformaldehydharz imprägnierten Dekorpapier ein nicht brennbarer Verbund hergestellt werden kann, welcher zudem eine geringe Gesamtdicke als auch ein geringes Gesamtgewicht aufweist. Dies wird insbesondere durch das Verpressen gemäß dem erfindungsgemäßen Verfahren erreicht.

In einer vorteilhaften Ausgestaltung der Erfindung ist vorgesehen, dass ein Glasfaservlies für die Trägermaterialschicht des Schichtstoffes verwendet wird. Durch die Verwendung eines Glasfaservlieses erhält die Trägermaterialschicht insbesondere verbesserte Eigenschaften hinsichtlich des Brandschutzes, da ein Entflammen durch deren Verwendung nicht einfach möglich ist.

In einer weiteren vorteilhaften Ausgestaltung der Erfindung ist vorgesehen, dass die Trägermaterialschicht des Schichtstoffes aus Quarzglasfasern besteht, da diese eine hohe chemische Beständigkeit aufweisen, eine hohe Erweichungstemperatur besitzen sowie zudem eine erhöhte Durchschlagsfestigkeit aufweisen. Dadurch wird die Trägermaterialschicht des Schichtstoffes nicht nur resistenter gegenüber einem Brand, sondern auch stabiler.

Eine vorteilhafte Ausgestaltung der Erfindung sieht weiter vor, dass die Trägermaterialschicht aus einem Wirrlagen-Vlies besteht. Bei einem Wirrlagen-Vlies können die Spinnfasern beziehungsweise die Filamente jede beliebige Richtung einnehmen, so dass aus dieser Gleichverteilung in allen Richtungen des Vliesstoffes eine erhöhte Stabilität resultiert.

In einer weiteren vorteilhaften Ausgestaltung der Erfindung wird, im zur Imprägnierung der Trägermaterialschicht verwendeten Kunstharz ein Crosslinker als Zusatzstoff verwendet. Durch die Verwendung eines Crosslinkers kann beim eingesetzten Kunstharz sowohl eine Erhöhung der Härte als auch der Zähigkeit und Flexibilität des erfindungsgemäßen Schichtstoffs erreicht werden.

Es können allgemein im zur Imprägnierung der Trägermaterialschicht verwendeten Kunstharz ein Metallhydroxid, Metallcarbonat, Titaniumoxid, kalzinierter Löss, Bariumsulfat, Magnesiumsulfat, Aluminiumsulfat, Zinkoxid, Kaolin, Chlorit, Diatomit, Feldspat, Glimmer, Nephelinsyenit, Pyrophyllit, Silizium, Talk, Wollastonit, Montmorillonit, Hektorit, Saponit, Calciumcarbonat, Magnesiumcarbonat, Aluminiumoxid, Eisenoxid, Magnesiumhydroxid und/oder Mikrokügelchen aus Glas als Füllstoff verwendet werden. Durch die Verwendung eines oder mehrerer der genannten Füllstoffe werden die Eigenschaften des zur Imprägnierung der Trägermaterialschicht verwendeten Kunstharzes hinsichtlich seines Einsatzes für den Brandschutz weiter verbessert, insbesondere derart, dass der Brennwert des Schichtstoffes reduziert wird und sich dies nachhaltig auf das allgemeine Brandverhalten auswirkt. Erfindungsgemäß werden im zur Imprägnierung des Vliesstoffes verwendeten Kunstharz Bariumsulfat, Magnesiumsulfat, Aluminiumsulfat, Calciumcarbonat, und/oder Magnesiumcarbonat als Füllstoff verwendet.

Gemäß einer bevorzugten Ausgestaltung des erfindungsgemäßen Schichtstoffes wird nur eine Trägermaterialschicht verwendet, da auf diese Weise sowohl Gesamtdicke als auch das Gewicht des Schichtstoffes gering gehalten werden können.

In einer weiteren vorteilhaften Ausgestaltung des erfindungsgemäßen Schichtstoffes ist vorgesehen, dass das zu imprägnierende Dekorpapier ein spezifisches Flächengewicht zwischen 22 g/m² und 220 g/m² aufweist. Durch die Verwendung eines besonders leichten Dekorpapiers ist es möglich, einen erfindungsgemäßen Schichtstoff auch in Bereichen einzusetzen, bei denen lediglich geringe Gewichte zum Einsatz kommen dürfen, wie beispielsweise bei der Beschichtung von Baustoffplatten. Wird hingegen ein hohes Gewicht des Schichtstoffes, beispielsweise für eine erhöhte Stabilität benötigt, so wird ein Dekorpapier mit einem hohen spezifischen Flächengewicht der mehrere übereinander angeordnete Lagen Dekorpapier verwendet.

Vorteilhafterweise weist das zu imprägnierende Dekorpapier des erfindungsgemäßen Schichtstoffes eine Porosität nach Gurley von weniger als 20 s/100ml gemäß ISO 5636/5 auf, da hierdurch die Aufnahme des verwendeten Kunstharzes zur Imprägnierung wesentlich verbessert werden kann.

In einer weiteren vorteilhaften Ausgestaltung des Schichtstoffes weist das zu imprägnierende Dekorpapier eine Glätte nach Bekk von mehr als 70 s/Bekk gemäß ISO 5627 auf, da hierdurch zum einen ein verbessertes optisches Erscheinungsbild realisiert wird und ferner eine verbesserte Vorbereitung der Oberfläche für eine optionale spätere Beschichtung mit einem weiteren Zusatzstoff erreicht werden kann.

In einer weiteren besonders vorteilhaften Ausgestaltung des erfindungsgemäßen Schichtstoffes weist das zu imprägnierende Dekorpapier eine Penetration von weniger als 5s gemäß ISO 5633 in Bezug auf das zur Imprägnierung verwendete Melaminformaldehydharz auf. Dadurch wird insbesondere die Aufnahme großer Mengen des Melaminformaldehydharzes ermöglicht, woraus eine Verbesserung der Brandschutzeigenschaften resultiert.

In einer vorteilhaften Ausgestaltung des erfindungsgemäßen Schichtstoffes ist ferner vorgesehen, dass das zu imprägnierende Dekorpapier im Blattbildungsprozess vorzugsweise aus Kurzfasern und/oder Langfasern hergestellt wird. Die Verwendung von Kurzfasern kann dann sinnvoll sein, falls an den herzustellenden Schichtstoff erhöhte Anforderungen bezüglich Stabilität bestehen, wobei hingegen Langfasern verwendet werden können, falls die Einarbeitung einer bestimmten Sollbruchstelle im Schichtstoff gewünscht ist.

In einer weiteren vorteilhaften Ausgestaltung der Erfindung ist vorgesehen, dass das zu imprägnierende Dekorpapier einen Ascheanteil von wenigstens 20 Gewichtsprozent enthält. Durch Verwendung eines hohen Ascheanteils für das zu imprägnierende Dekorpapier wird der Anteil der brennbaren Bestandteile im Dekorpapier reduziert und somit die hinsichtlich des Brennwertes bestehenden Eigenschaften gemindert, wodurch die gesamte Feuerbeständigkeit des Schichtstoffes weiter erhöht werden kann.

In einer vorteilhaften Ausgestaltung des erfindungsgemäßen Schichtstoffes wird zur Färbung und/oder zur Bedruckung des zur imprägnierenden Dekorpapiers eine aus anorganischen Stoffen bestehende Farbe verwendet. Dies ist insbesondere für ein verbessertes Brandverhalten vorteilhaft, da durch die Verwendung von aus anorganischen Stoffen bestehender Farbe der Schichtstoff weiter resistent gegenüber Feuer gemacht wird.

Vorteilhafterweise wird zur Imprägnierung des Dekorpapiers des erfindungsgemäßen Schichtstoffes beim verwendeten Melaminformaldehydharz Titanoxid, Talkum oder dergleichen als Füllstoff verwendet. Derartige Stoffe sind thermisch stabil und weisen einen besonders hohen Schmelzpunkt auf, so dass dies zur weiteren Verbesserung des Brandverhaltens genutzt werden kann.

In einer vorteilhaften Ausgestaltung des erfindungsgemäßen Schichtstoffes ist die der imprägnierten Trägermaterialschicht abgewandte Oberfläche der Dekormaterialschicht mit einem Flammschutzmittel versehen. Durch die ' Verwendung eines Flammschutzmittels besteht ein zusätzlicher' Schutz der feuerresistent ausgebildeten Dekormaterialschicht, insbesondere da es so bei einem Brandfall angreifenden Flammen erschwert wird, überhaupt mit der Dekormaterialschicht direkt in Berührung zukommen.

Die zuvor beschriebene Nachteile des Standes der Technik werden durch ein Verfahren zur Herstellung eines Schichtstoffes, welcher den Anforderungen der Baustoffklasse A1 gemäß DIN 4102-1 beziehungsweise EN 13501 genügt, beseitigt, welches nicht Gegenstand der Ansprüche ist, wobei wenigstens eine Trägermaterialschicht aus einem nicht brennbaren, mit einem Kunstharz imprägnierten Vliesstoff mit wenigstens einer Dekormaterialschicht aus einem mit Melaminformaldehydharz imprägnierten, gegebenenfalls gefärbten und/oder bedruckten Dekorpapier derart zu einem Verbund verpresst wird, dass der aus dem Verpressen resultierende Schichtstoff eine Gesamtdicke von weniger als 2,2 mm, vorzugsweise in einem Bereich von 0,3 mm bis unter 2,2 mm, besonders bevorzugt in einem Bereich von 0,5 mm bis 1,3 mm, und einem Brennwert von weniger als 2 MJ/kg aufweist.

Eine Ausgestaltung des Verfahrens sieht dabei vor, dass die Trägermaterialschicht vor dem Imprägnieren mit dem Kunstharz geschliffen wird. Auf diese Weise wird eine verbesserte Aufnahme des zu imprägnierenden Kunstharzes erzielt.

Gemäß einer weiteren Ausgestaltung des Verfahrens erfolgt nach dem Verpressen der imprägnierten Trägermaterialschicht mit der imprägnierten Dekormaterialschicht das Aufbringen eines Flammschutzmittels auf die der Trägermaterialschicht abgewandten Seite der Dekormaterialschicht. Dadurch kann ein verbessertes Brandverhalten erzielt werden, da durch das Flammschutzmittel im Brandfall verhindert wird, dass Flammen überhaupt zur Dekormaterialschicht gelangen.

Eine weitere Ausgestaltung des Verfahrens sieht vor, dass bei dem Verpressen eine Vermengung des in der Trägermaterialschicht imprägnierten Kunstharzes und des in der Dekormaterialschicht imprägnierten Melaminformaldehydharzes erfolgt. Durch die Vermengung der beiden Harze, welche daraus resultiert, dass diese Harze auch im Grenzbereich der beiden Schichten vorhanden sind, erfolgt eine weitere Verbesserung des Schichtstoffes hinsichtlich seines Brandverhaltens.

Eine weitere Ausgestaltung des Verfahrens sieht vor, dass das Verpressen unter Temperatur und hohem Flächendruck mittels einer Ein- oder Mehretagenpresse oder einer kontinuierlichen Doppelbandpresse mit oder ohne Rückkühlung erfolgt. Durch die Verwendung einer Mehretagenpresse kann die Effizienz zur Herstellung erhöht werden, wobei hingegen bei Verwendung einer Einetagenpresse auch die Fertigung einer kleinen Stückzahl ermöglicht wird. Durch das Vorhandensein einer Rückkühlung können insbesondere die Eigenschaften des erfindungsgemäßen Schichtstoffes mit dem Verfahren weiter verbessert werden.

Weitere Einzelheiten, Merkmale und Vorteile der Erfindung werden nachfolgend anhand der in den Figuren der Zeichnungen dargestellten Ausführungsbeispiele näher erläutert.

Dabei zeigen:
- Fig. 1: in schematischer Schnittansicht ein Ausführungsbeispiel eines erfindungsgemäßen Schichtstoffes;
- Fig. 2a bis 2d: in schematischen Schnittansichten ein Ausführungsbeispiel eines Verfahrens zur Herstellung eines erfindungsgemäßen Schichtstoffes; und
- Fig. 3: in schematischer Schnittansicht ein weiteres Ausführungsbeispiel eines erfindungsgemäßen Schichtstoffes.

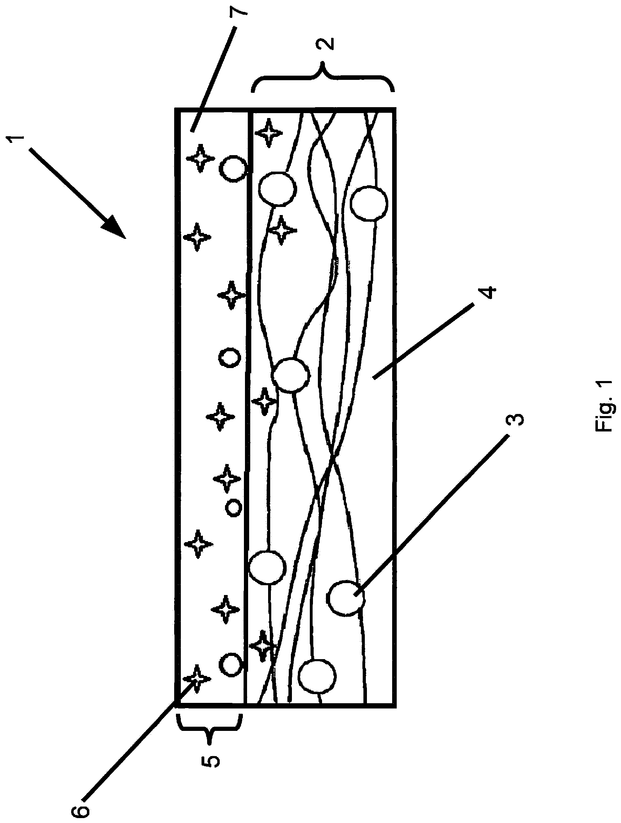

Fig. 1 zeigt in einer schematischen Schnittansicht einen Schichtstoff 1. Der Schichtstoff 1 besteht aus einem Verbund aus einer Trägermaterialschicht 2 und einer Dekormaterialschicht 5. Dabei besteht die Trägermaterialschicht 2 aus einem nicht brennbaren, mit einem Kunstharz 3 imprägnierten Vliesstoff 4. Die Dekormaterialschicht 5 besteht hingegen aus einem mit Melaminformaldehydharz 6 imprägnierten Dekorpapier 7. Weiterhin ist aus der Figur 1 zu erkennen, dass bei einem nach dem erfindungsgemäßen Verfahren hergestellten Schichtstoff eine Vermengung des in der Trägermaterialschicht 2 imprägnierten Kunstharzes 3 und des in der Dekormaterialschicht 5 imprägnierten Melaminformaldehydharzes 6 erfolgte, woraus eine weitere Verbesserung des Schichtstoffes 1 hinsichtlich seines Brandverhaltens resultiert.

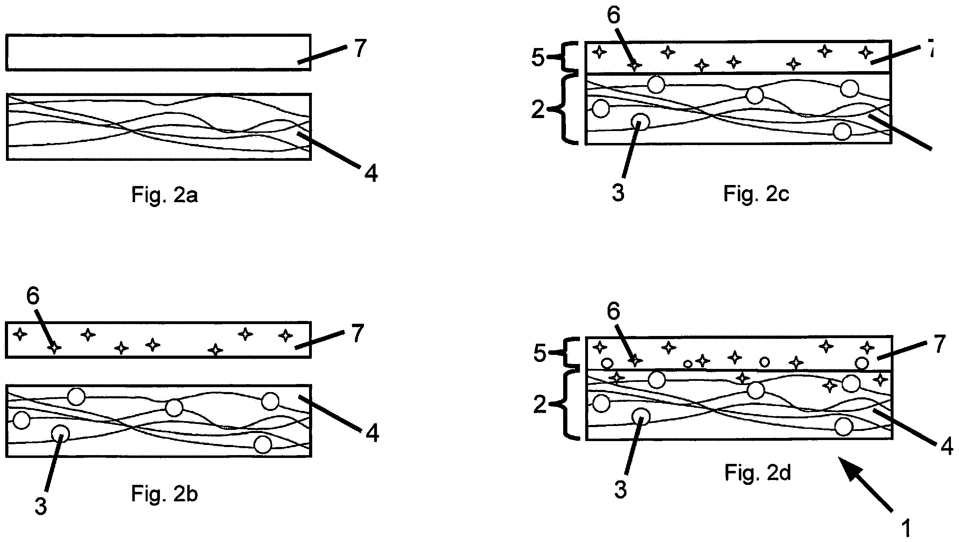

Fig. 2a (erster Verfahrensschritt) zeigt in einer schematischen Schnittansicht einen nicht brennbaren Vliesstoff 4 für eine Trägermaterialschicht sowie ein gegebenenfalls gefärbtes und/oder bedrucktes Dekorpapier 7 für eine Dekormaterialschicht. Weder der Vliesstoff 4 noch das gegebenenfalls gefärbte und/oder bedruckte Dekorpapier 7 sind in diesem Verfahrensschritt mit einem Kunstharz imprägniert und auch nicht miteinander verbunden.

Fig. 2b (zweiter Verfahrensschritt) zeigt in einer schematischen Schnittansicht einen nicht brennbaren, nun mit einem Kunstharz 3 imprägnierten Vliesstoff 4 sowie ein gegebenenfalls gefärbtes und/oder bedrucktes Dekorpapier 7, welches mit einem Melaminformaldehydharz 6 imprägniert wurde. Die Imprägnierung der beiden Einzelbestandteile, welche für einen aus einem Verbund bestehenden Schichtstoff benötigt werden, kann dabei sowohl sequenziell als auch parallel erfolgen. Ferner ist es denkbar, dass eine bereits vorkonfektionierte Lage eines mit einem Kunstharz 3 imprägnierten Vliesstoffes 4 und/oder eines mit einem Melaminformaldehydharz 6 imprägnierten, gegebenenfalls gefärbten und/oder bedruckten Dekorpapiers 7 verwendet wird.

Fig. 2c (dritter Verfahrensschritt) zeigt in einer schematischen Schnittansicht wie ein aus einem nicht brennbaren, mit einem Kunstharz 3 imprägnierter Vliesstoff 4 mit einem mit Melaminformaldehydharz 6 imprägnierten, gegebenenfalls gefärbten und/oder bedruckten Dekorpapier 7 zu einem Verbund verpresst wird. Eine Verpressung erfolgt dabei unter Temperatur und hohem Flächendruck mittels einer Ein- oder Mehretagenpresse oder einer kontinuierlichen Doppelbandpresse mit oder ohne Rückkühlung. Durch eine derartige Verpressung kann der aus Fig. 1 bekannte Schichtstoff hergestellt werden.

Fig. 2d (vierter Verfahrensschritt) zeigt in einer schematischen Schnittansicht einen erfindungsgemäßen Schichtstoff 1 in Form eines Verbundes, welcher aus der Verpressung der Trägermaterialschicht 2 aus einem nicht brennbaren, mit einem Kunstharz 3 imprägnierten Vliesstoff 4 und einer Dekormaterialschicht 5 aus einem mit Melaminformaldehydharz 6 imprägnierten, gegebenenfalls gefärbten und/oder bedruckten Dekorpapier 7 resultiert. In Fig. 2d ist ferner zu erkennen, dass bei dem Verpressen der beiden Materialschichten eine Vermengung des in der Trägermaterialschicht 2 imprägnierten Kunstharzes 3 und des in der Dekormaterialschicht 5 imprägnierten Melaminformaldehydharzes 6 insbesondere im Grenzbereich der beiden Materialschichten erfolgte. Durch eine derartige Vermengung der verschiedenen Harze resultiert eine weitere Verbesserung des Schichtstoffes 1 hinsichtlich seines Brandverhaltens.

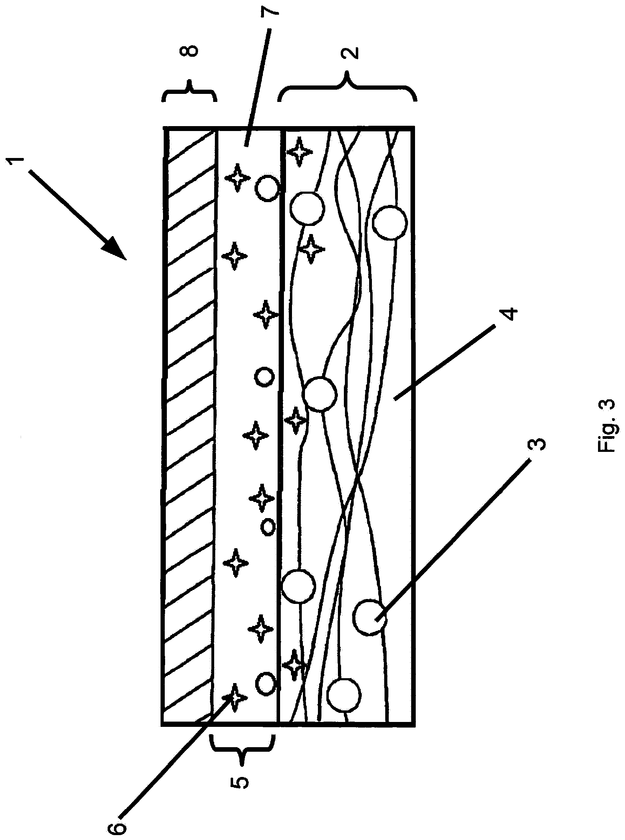

Fig. 3 zeigt in einer schematischen Schnittansicht einen erfindungsgemäßen Schichtstoff, bei welchem auf der der imprägnierten Trägermaterialschicht 2 abgewandten Oberfläche der Dekormaterialschicht 5 ein Flammschutzmittel 8 aufgetragen ist. Dies dient insbesondere der weiteren Verbesserung des erfindungsgemäßen Schichtstoffes 1 hinsichtlich des Brandschutzes.

Die in den Figuren der Zeichnung dargestellten Ausführungsbeispiele und die im Zusammenhang mit diesen erläuterten Ausführungsbeispiele dienen lediglich einer Erläuterung der Erfindung und sind für die nicht beschränkend.

### Bezuaszeichenliste

- 1: Schichtstoff
- 2: Trägermaterialschicht
- 3: Kunstharz
- 4: Vliesstoff
- 5: Dekormaterialschicht
- 6: Melaminformaldehydharz
- 7: Dekorpapier
- 8: Flammschutzmittel

## Patentansprüche

1. Schichtstoff (1)
eigens konzipiert zur Herstellung einer Baustoffplatte, welche den Anforderungen der Baustoffklasse A1 gemäß DIN 4102-1 beziehungsweise EN 13501 genügt, durch Beschichtung der Baustoffplatte mit dem Schichtstoff (1),
wobei der Schichtstoff (1) aus einem Verbund aus
einer Trägermaterialschicht (2) aus einem nicht brennbaren, mit einem Kunstharz (3) imprägnierten Vliesstoff (4), wobei der Vliesstoff (4) ein Glasfaservlies ist,
und
wenigstens einer Dekormaterialschicht (5) aus einem mit einem Kunstharz imprägnierten Dekorpapier (7),
besteht,
und durch Verpressen der. Trägermaterialschicht (2) mit der Dekormaterialschicht (5) hergestellt ist,
**dadurch gekennzeichnet, dass**
der Schichtstoff (1) eine Gesamtdicke in einem Bereich von 0,3 mm bis unter 2,2 mm und einen Brennwert von weniger als 2 MJ/kg gemäß ISO 1716 aufweist,
im zur Imprägnierung des Vliesstoffes (4) der Trägermaterialschicht (2) verwendeten Kunstharz (3) Bariumsulfat, Magnesiumsulfat, Aluminiumsulfat, Calciumcarbonat, und/oder Magnesiumcarbonat als Füllstoff verwendet sind, und
zur Imprägnierung des Dekorpapiers (7) der Dekormaterialschicht (5) als Kunstharz ein Melaminformaldehydharz (6) verwendet ist.

2. Schichtstoff (1) nach Anspruch 1, **dadurch gekennzeichnet, dass** dieser eine Gesamtdicke in einem Bereich von 0,5 mm bis 1,3 mm aufweist.

3. Schichtstoff (1) nach Anspruch 1 oder Anspruch 2, **dadurch gekennzeichnet, dass** im zur Imprägnierung des Vliesstoffs (4) der Trägermaterialschicht (2) verwendeten Kunstharz (3) ein Crosslinker als Zusatzstoff verwendet ist.

4. Schichtstoff (1) nach einem der Ansprüche 1 bis 3, **dadurch gekennzeichnet, dass** nur eine Trägermaterialschicht (2) verwendet ist.

5. Schichtstoff (1) nach einem der Ansprüche 1 bis 4, **dadurch gekennzeichnet, dass** der Vliesstoff (4) der Trägermaterialschicht (2) aus Quarzglasfasern besteht.

6. Schichtstoff (1) nach einem der Ansprüche 1 bis 5, **dadurch gekennzeichnet, dass** der Vliesstoff (4) der Trägermaterialschicht (2) ein Wirrlagen-Vlies ist.

7. Schichtstoff nach einem der Ansprüche 1 bis 6, **dadurch gekennzeichnet, dass** der Vliesstoff (4) der Trägermaterialschicht (2) vor dem Imprägnieren mit dem Kunstharz (3) geschliffen ist.

8. Schichtstoff (1) nach einem der Ansprüche 1 bis 7, **dadurch gekennzeichnet, dass** das Dekorpapier (7) der Dekormaterialschicht (5) gefärbt und/oder bedruckt ist.

9. Schichtstoff (1) nach Anspruch 8, **dadurch gekennzeichnet, dass** zur Färbung und/oder Bedruckung des zu imprägnierenden Dekorpapiers (7) der Dekormaterialschicht (5) eine aus anorganischen Stoffen bestehende Farbe verwendet ist.

10. Schichtstoff (1) nach einem der Ansprüche 1 bis 9, **dadurch gekennzeichnet, dass** das zu imprägnierende Dekorpapier (7) der Dekormaterialschicht (5) ein spezifisches Flächengewicht zwischen 22g/m² und 220g/m² aufweist.

11. Schichtstoff (1) nach einem der Ansprüche 1 bis 10, **dadurch gekennzeichnet, dass** das zu imprägnierende Dekorpapier (7) der Dekormaterialschicht (5) eine Porosität nach Gurley von weniger als 20 s/100ml gemäß ISO 5636/5 aufweist.

12. Schichtstoff (1) nach einem der Ansprüche 1 bis 11, **dadurch gekennzeichnet, dass** das zu imprägnierende Dekorpapier (7) der Dekormaterialschicht (5) eine Glätte nach Bekk von mehr als 70 s/Bekk gemäß ISO 5627 aufweist.

13. Schichtstoff (1) nach einem der Ansprüche 1 bis 12, **dadurch gekennzeichnet, dass** das zu imprägnierende Dekorpapier (7) der Dekormaterialschicht (5) eine Penetration von weniger als 5s gemäß ISO 5633 in Bezug auf das zur Imprägnierung verwendete Melaminformaldehydharz (6) aufweist.

14. Schichtstoff (1) nach einem der Ansprüche 1 bis 13, **dadurch gekennzeichnet, dass** das zu imprägnierende Dekorpapier (7) der Dekormaterialschicht (5) im Blattbildungsprozess aus Kurzfasern und Langfasem hergestellt ist.

15. Schichtstoff (1) nach einem der Ansprüche 1 bis 14, **dadurch gekennzeichnet, dass** das zu imprägnierende Dekorpapier (7) der Dekormaterialschicht (5) einen Ascheanteil von wenigstens 20 Gew.-% enthält.

16. Schichtstoff (1) nach einem der Ansprüche 1 bis 15, **dadurch gekennzeichnet, dass** im zur Imprägnierung des Dekorpapiers (7) der Dekormaterialschicht (5) verwendeten Melaminformaldehydharz (6) Titanoxid oder Talkum als Füllstoff verwendet ist.

17. Schichtstoff (1) nach einem der Ansprüche 1 bis 16, **dadurch gekennzeichnet, dass** die der imprägnierten Trägermaterialschicht (2) abgewandte Oberfläche der Dekormaterialschicht (5) mit einem Flammschutzmittel (8) versehen ist.

18. Schichtstoff (1) nach Anspruch 17, **dadurch gekennzeichnet, dass** das Flammschutzmittel (8) nach dem Verpressen der imprägnierten Trägermaterialschicht (2) mit der imprägnierten Dekormaterialschicht (5) auf die der Trägermaterialschicht (2) abgewandte Seite der Dekormaterialschicht (5) aufgebracht ist.

## Claims

1. A laminate (1),
specially designed for manufacturing a construction panel, which meets the requirements of the building material class A1 according to DIN 4102-1 or EN 13501, by coating the construction panel with the laminate (1),
the laminate (1) consisting of a combination of
a carrier material layer (2) made of a non-flammable non-woven fabric (4) impregnated with a synthetic resin (3), whereby the non-woven fabric (4) is a glass fiber non-woven fabric,
and
at least one decorative material layer (5) made of a decorative paper (7) that is impregnated with a resin,
and is produced by pressing the carrier material layer (2) with the decorative material layer (5),
**characterized in that**
the laminate (1) comprises a total thickness between 0.3 mm and beneath 2.2 mm and a calorific value of less than 2 MJ/kg according to ISO 1716,
barium sulfate, magnesium sulfate, aluminum sulfate, calcium carbonate, and/or magnesium carbonate, are used as filling material in the synthetic resin (3) which is used for impregnating the non-woven fabric (4) of the carrier material layer (2), and
for impregnation of the decorative paper (7) of the decorative material layer (5) as resin a melamine formaldehyde resin (6) is used.

2. A laminate (1) according to claim 1, **characterized in that** the laminate (1) comprises a total thickness between 0.5 mm and 1.3 mm.

3. A laminate (1) according to claim 1 or claim 2, **characterized in that** a crosslinker is used as additive in the synthetic resin (3) that is used for impregnating the non-woven fabric (4) of the carrier material layer (2).

4. A laminate (1) according to one of the claims 1 to 3, **characterized in that** only one carrier material layer (2) is used.

5. A laminate (1) according to one of the claims 1 to 4, **characterized in that** the non-woven fabric (4) of the carrier material layer (2) consists of quartz glass fibers.

6. A laminate (1) according to one of the claims 1 to 5, **characterized in that** the non-woven fabric (4) of the carrier material layer (2) is a random orientation fleece.

7. A laminate (1) according to one of the claims 1 to 6, **characterized in that** the non-woven fabric (4) of the carrier material layer (2) is ground before impregnation with the synthetic resin (3).

8. A laminate (1) according to one of the claims 1 to 7, **characterized in that** the decorative paper (7) of the decorative material layer (5) dyed and/or printed.

9. A laminate (1) according to claim 8, **characterized in that** a dye consisting of inorganic substances will be used for dyeing and/or printing the decorative paper (7) of the decorative material layer (5) to be impregnated.

10. A laminate (1) according to one of the claims 1 to 9, **characterized in that** the decorative paper (7) of the decorative material layer (5) to be impregnated comprises a specific surface weight comprised between 22 g/m² and 220 g/m².

11. A laminate (1) according to one of the claims 1 to 10, **characterized in that** the decorative paper (7) of the decorative material layer (5) to be impregnated comprises a porosity according to Gurley of less than 20 s/100 ml according to ISO 5636/5.

12. A laminate (1) according to one of the claims 1 to 11, **characterized in that** the decorative paper (7) of the decorative material layer (5) to be impregnated comprises a smoothness of Bekk of more than 70 s/Bekk according to ISO 5627.

13. A laminate (1) according to one of the claims 1 to 12, **characterized in that** the decorative paper (7) of the decorative material layer (5) to be impregnated comprises a penetration of less than 5s according to ISO 5633 with respect to the melamine formaldehyde resin (6) used for the impregnation.

14. A laminate (1) according to one of the claims 1 to 13, **characterized in that** the decorative paper (7) of the decorative material layer (5) to be impregnated is produced from short fibers and long fibers in the sheet forming process.

15. A laminate (1) according to one of the claims 1 to 14, **characterized in that** the decorative paper (7) of the decorative material layer (5) to be impregnated comprises an ash content of at least 20 % by weight.

16. A laminate (1) according to one of the claims 1 to 15, **characterized in that** titanium oxide or talcum is used as filling material in the melamine formaldehyde resin (6) used for impregnating the decorative paper (7) of the decorative material layer (5).

17. A laminate (1) according to one of the claims 1 to 16, **characterized in that** the surface of the decorative material layer (5) which is facing away from the impregnated carrier material layer(2) is provided with a flame retardant (8).

18. A laminate (1) according to claim 17, **characterized in that** the flame retardant (8) is applied to the side of the decorative material layer (5) facing away from the carrier material layer (2) after the impregnated carrier material layer (2) has been pressed together with the impregnated decorative material layer (5).

## Revendications

1. Stratifié (1),
spécialement conçu pour la fabrication d'un panneau de construction, qui répond aux exigences de la classe de matériaux de construction A1 selon la norme DIN 4102-1 ou EN 13501, en revêtant le panneau de construction avec le stratifié (1), le stratifié (1)
consistant en une combinaison de
une couche de matériau support (2) constituée d'un tissu non tissé ininflammable (4) imprégné d'une résine synthétique (3), le tissu non tissé (4) étant un tissu non tissé en fibres de verre,
et
au moins une couche de matériau décoratif (5) constituée d'un papier décoratif (7) qui est imprégné d'une résine,
et est produit en pressant la couche de matériau de support (2) avec la couche de matériau décoratif (5),
**caractérisé en ce que**
le stratifié (1) présente une épaisseur totale comprise entre 0,3 mm et moins de 2,2 mm et un pouvoir calorifique inférieur à 2 MJ/kg selon la norme ISO 1716,
du sulfate de baryum, du sulfate de magnésium, du sulfate d'aluminium, du carbonate de calcium et/ou du carbonate de magnésium, sont utilisés comme matériau de remplissage dans la résine synthétique (3) qui est utilisée pour imprégner le tissu non tissé (4) de la couche de matériau support (2), et
pour l'imprégnation du papier décoratif (7) de la couche de matériau décoratif (5) en tant que résine, on utilise une résine de mélamine-formaldéhyde (6).

2. Stratifié (1) selon la revendication 1, **caractérisé en ce que** le stratifié (1) comprend une épaisseur totale comprise entre 0,5 mm et 1,3 mm.

3. Stratifié (1) selon la revendication 1 ou la revendication 2, **caractérisé en ce qu'**un réticulant est utilisé comme additif dans la résine synthétique (3) qui est utilisée pour imprégner le tissu non tissé (4) de la couche de matériau support (2).

4. Stratifié (1) selon l'une des revendications 1 à 3, **caractérisé en ce qu'**une seule couche de matériau de support (2) est utilisée.

5. Stratifié (1) selon l'une des revendications 1 à 4, **caractérisé en ce que** le tissu non tissé (4) de la couche de matériau support (2) est constitué de fibres de verre de quartz.

6. Stratifié (1) selon l'une des revendications 1 à 5, **caractérisé en ce que** le tissu non tissé (4) de la couche de matériau support (2) est un non-tissé à orientation aléatoire.

7. Stratifié (1) selon l'une des revendications 1 à 6, **caractérisé en ce que** le tissu non tissé (4) de la couche de matériau support (2) est broyé avant l'imprégnation par la résine synthétique (3).

8. Stratifié (1) selon l'une des revendications 1 à 7, **caractérisé en ce que** le papier décoratif (7) de la couche de matériau décoratif (5) est teinté et/ou imprimé.

9. Stratifié (1) selon la revendication 8, **caractérisé en ce qu'**un colorant constitué de substances inorganiques sera utilisé pour teindre et/ou imprimer le papier décoratif (7) de la couche de matériau décoratif (5) à imprégner.

10. Stratifié (1) selon l'une des revendications 1 à 9, **caractérisé en ce que** le papier décoratif (7) de la couche de matériau décoratif (5) à imprégner comprend un poids spécifique de surface compris entre 22 g/m² et 220 g/m².

11. Stratifié (1) selon l'une des revendications 1 à 10, **caractérisé en ce que** le papier décoratif (7) de la couche de matériau décoratif (5) à imprégner présente une porosité selon Gurley inférieure à 20 s/100 ml selon ISO 5636/5.

12. Stratifié (1) selon l'une des revendications 1 à 11, **caractérisé en ce que** le papier décoratif (7) de la couche de matériau décoratif (5) à imprégner comprend un lissé de Bekk de plus de 70 s/Bekk selon ISO 5627.

13. Stratifié (1) selon l'une des revendications 1 à 12, **caractérisé en ce que** le papier décoratif (7) de la couche de matériau décoratif (5) à imprégner présente une pénétration inférieure à 5s selon la norme ISO 5633 par rapport à la résine mélamine-formaldéhyde (6) utilisée pour l'imprégnation.

14. Stratifié (1) selon l'une des revendications 1 à 13, **caractérisé en ce que** le papier décoratif (7) de la couche de matériau décoratif (5) à imprégner est produit à partir de fibres courtes et de fibres longues dans le processus de formation de la feuille.

15. Stratifié (1) selon l'une des revendications 1 à 14, **caractérisé en ce que** le papier décoratif (7) de la couche de matériau décoratif (5) à imprégner comprend une teneur en cendres d'au moins 20 % en poids.

16. Stratifié (1) selon l'une des revendications 1 à 15, **caractérisé en ce que** l'oxyde de titane ou le talc est utilisé comme matériau de remplissage dans la résine de mélamine-formaldéhyde (6) utilisée pour imprégner le papier décoratif (7) de la couche de matériau décoratif (5).

17. Stratifié (1) selon l'une des revendications 1 à 16, **caractérisé en ce que** la surface de la couche de matériau décoratif (5) qui est opposée à la couche de matériau de support imprégné (2) est pourvue d'un agent ignifuge (8).

18. Stratifié (1) selon la revendication 17, **caractérisé en ce que** le retardateur de flamme (8) est appliqué sur le côté de la couche de matériau décoratif (5) opposé à la couche de matériau de support (2) après que la couche de matériau de support imprégnée (2) a été pressée ensemble avec la couche de matériau décoratif imprégnée (5).
